# EUROPEAN PATENT SPECIFICATION

(11) **EP 0 370 357 B1**
(45) Date of publication and mention of the grant of the patent: **22.06.1994**
(21) Application number: 89121019.7
(22) Date of filing: 13.11.1989
(51) Int. Cl.: C07C 255/61, C07C 253/30

(54) **Process for producing 3-iminonitriles**
Verfahren zur Herstellung von 3-Iminonitrilen
Procédé pour la production de 3-iminonitriles

(30) Priority: 14.11.1988 JP 287039/88
(43) Date of publication of application: 30.05.1990
(73) Proprietor: FUJI PHOTO FILM CO., LTD., Kanagawa (JP)
(72) Inventor: Taniguchi, Masato, Minami Ashigara-shi Kanagawa (JP); Sato, Tadahisa, Minami Ashigara-shi Kanagawa (JP); Mizukawa, Yuki, Minami Ashigara-shi Kanagawa (JP)
(74) Representative: Grünecker, Kinkeldey, Stockmair & Schwanhäusser Anwaltssozietät

(56) References cited:
- AT-B- 375 067
- US-A- 3 290 355
- JOURNAL OF ORGANIC CHEMISTRY, vol. 16, no. 2, February 1951, pages 165-172; G.A. REYNOLDS: "Condensations of nitriles having alpha-hydrogen to form beta-iminonitriles, cyclic trimers, and substituted 2-hydroxy-4-aminopyridines"
- JOURNAL OF THE AMERICAN CHEMICAL SOCIETY, vol. 64, no. 1, 8th January 1942, pages 150-154; H. ADKINS, "Hydrogenation of beta-iminonitriles"
- pages 150-154; H. ADKINS: "Hydrogenation of beta-iminonitriles"

## Description

This invention relates to a process for producing 3-iminonitriles and, more particularly, to a process for producing 3-iminonitriles useful as intermediates for synthesizing photographic couplers or medicines.

3-Iminonitriles are useful as intermediates for synthesizing photographic couplers or medicines. For example, 3-iminobutyronitrile which is a 3-iminonitrile is used in JP-A-59-171956 (the term "JP-A" as used herein means an "unexamined published Japanese patent application") corresponding to US-A-4,540,654 as a starting material for synthesizing 1H-pyrazolo[1,5-b]-1,2,4-triazoles useful as photographic magenta couplers.

As to synthesis of the 3-iminonitriles, descriptions are given in Journal of the American Chemical Society, 64, 150 (1942), FR-A-1,377,891, Canadian Journal of Chemistry, 43, 332 (1965), and AT-A-375,067, disclosing, for example, the following synthesizing processes: 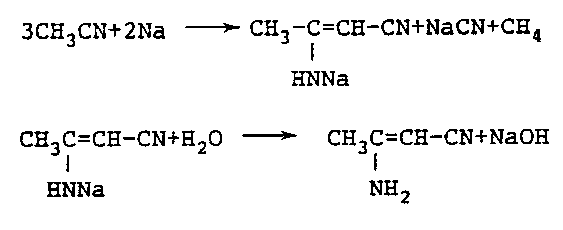
However, these processes result in the undesired production of poisonous sodium prussiate. In addition, in actual production, a step of removing the sodium prussiate is inevitably involved, and therefore the processes involve an additional problem in that the production costs of the 3-iminonitriles becomes seriously high.

It is therefore the object underlying the present invention to overcome these drawbacks in the known processes for producing 3-iminonitriles, in particular to provide 3-iminonitriles with producing substantially no sodium prussiate and, therefore, to synthesize 3-iminonitriles stably and inexpensively.

According to the present invention this is attained with a process for producing a 3-iminonitrile represented by the following general formula (II): 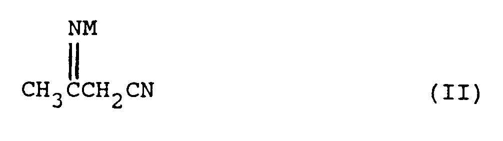
wherein M represents an alkali metal, which comprises reacting a compound of the formula (I):

CH₃CN (I)

with an alkali metal hydride.

In addition, the present invention provides a process for producing a 3-iminonitrile represented by the general formula (III): 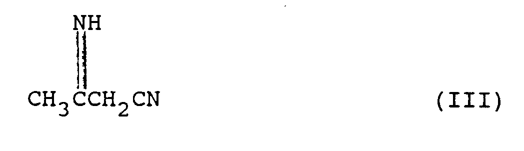
which comprises mixing the compound represented by the general formula 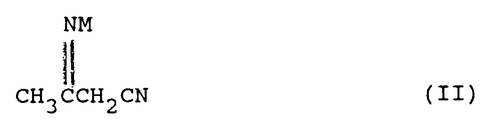
wherein M represents an alkali metal, with a protic solvent, wherein said compound represented by the general formula (II) is obtained by reacting a compound represented by the formula (I):

CH₃CN (I)

with an alkali metal hydride.

In the present invention, compounds represented by the general formula (III) are considered to be isomerized to 3-aminocrotononitriles.

An embodiment of the present invention is now described in detail below.

Synthesis steps of the present invention are shown by the following process (1): 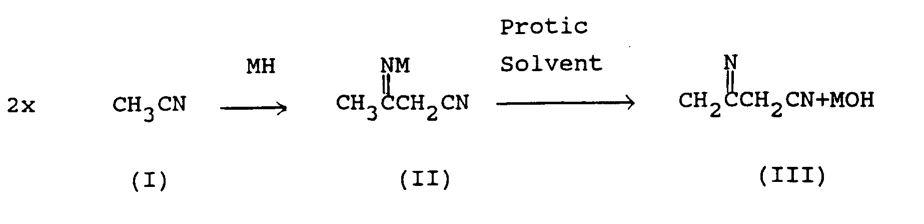
wherein M in the general formulae (II) and (III) are the same as defined hereinbefore.

The compound represented by the formula (I) is commercially available or the compound may be readily synthesized according to known processes as described in, for example, "Shin Jikken Kagaku Koza", Vol. 14, pp. 1428 to 1484 (1978).

Alkali metal hydrides are preferably used in an amount of from 0.5 to 5.0 molar equivalents, more preferably 0.5 to 1.1 molar equivalents, based on the compound represented by the formula (I).

Preferable metal hydrides include lithium hydride, sodium hydride and potassium hydride, with sodium hydride being particularly preferred.

As a reaction solvent to produce the compound of the general formula (II), any of commonly known aprotic solvents with high or low polarity may properly be selected and used alone or as a mixture thereof.

The amount of the solvent is not particularly limited, but it is preferably used in an amount of from 2 to 10 times as much as the compound of the formula (I) by weight.

The reaction solvent is preferably an amide series solvent (e.g., N,N-dimethylformamide or N,N-dimethylamide), an ether series solvent (e.g., tetrahydrofuran or dioxane), an aromatic hydrocarbon series solvent (e.g., benzene, toluene or xylene) or an aliphatic hydrocarbon series solvent (e.g., hexane or octane).

A reaction temperature ranging from 0°C to 200°C is preferred, with a range of from 20°C to 150°C being more preferred.

The reaction time is preferably 30 min to 10 h with 1 h to 5 h being more preferable. However, since the reaction time varies depending upon, e.g., the scale of the reaction it is not limited to the above-described range.

The reaction can be conducted under atmospheric pressure.

Compounds represented by the general formula (III) can be obtained by mixing the compound obtained by the above-described reaction and represented by the general formula (II) with a protic solvent such as water. The compound of formula (II) need not be isolated from the a protic solvent used in the above reaction before mixing with a protic solvent. The protic solvent is preferably used in an amount of at least 2 times as much as the amount of alkali metal hydride used in molar ratio, more preferably in an amount 3 to 5 times as much as the compound represented by the formula (I) by weight. The mixing procedure is conducted at a temperature of preferably from 0°C to room temperature under atmospheric pressure.

The 3-iminonitriles obtained according to process (1) described above can be separated from the reaction solution in a conventional manner but, if necessary, they may be used as the starting materials for the subsequent reaction without separation.

As a suitable isolating means, for example, recrystallization, solvent extraction, filtration, column chromatography and thin layer chromatography may be used independently or in a proper combination.

The present invention is now illustrated in greater detail by reference to the following examples. Unless otherwise indicated, all percents, ratios, parts, etc. are by weight.

### EXAMPLE 1

### Synthesis of illustrative compound (1)

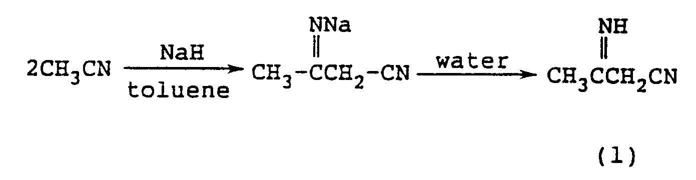

9.6 g (2.39×10⁻¹ mol) of sodium hydride (60% dispersion in mineral oil) were added to 75 ml of toluene and, under heat-refluxing and stirring, 25 ml (4.79×10⁻¹ mol) of acetonitrile were dropwise added thereto. Refluxing under heat was continued for an additional 3 h. Thereafter, a precipitate was collected by filtration, and the precipitate was dispersed in diethyl ether, followed by adding water thereto. Then, the organic phase was separated and dried over Glauber's salt, and diethyl ether was distilled off under reduced pressure to obtain 14.5 g of crystals containing the illustrative compound (1).

### EXAMPLE 2

The synthesis of illustrative compound (1) as well as the preparation of 5-amino-4-chloro-3-methyl-1H-pyrazole hydrochloride (B) which is useful as an intermediate for a photographic coupler from the resulting compound (1) are shown below. 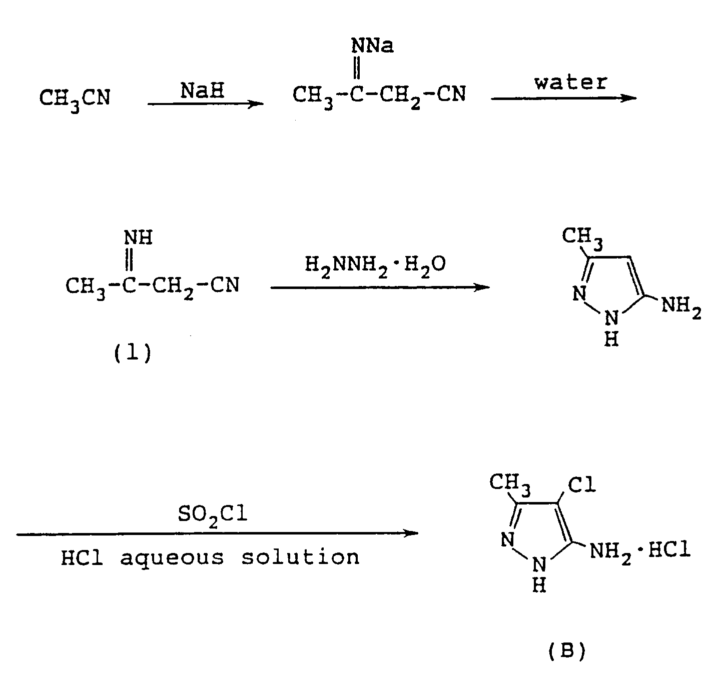
115 g (3.00 mol) of sodium hydride (62.5% dispersion in mineral oil) were added to 922 ml of acetonitrile, and the resulting mixture was stirred while heating at 60 to 70°C for 2 h. Thereafter, 300 ml of water and 200 ml of hexane were added to the mixture at a temperature of 30 to 40°C. The reaction mixture was separated into three layers, and the intermediate layer was isolated and acetonitrile was distilled off therefrom under reduced pressure. 225 g (3.60 mol) of 80% hydrazine hydrate were added to the residue, and the mixture was heated with stirring at 80°C for 1 h and then at 110 to 115°C for 2 h. After allowing to cool, 175 ml of acetone were added to the mixture. After heating the mixture at 60°C for 1 h while stirring, 40 ml of toluene were added thereto, and the solvent was distilled off under reduced pressure.

420 ml of 36% aqueous hydrochloric acid and 80 ml of water were added to the residue, and 361 ml of sulfuryl chloride were added dropwise thereto over 1 h while maintaining the reaction system at a temperature of 30°C or below, during which time crystals precipitated from the reaction system. Then, the reaction mixture was stirred for 4 h while maintaining the temperature at 20°C or below. Thereafter, the generated SO₂ was removed by an aspirator for 2 h under reduced pressure. The pressure of the reaction system was allowed to raise to atmospheric pressure, and 300 ml of acetone were added thereto, followed by stirring at 6°C for 2 h. The filter cake obtained by filtering the reaction mixture was washed with 300 ml of acetone and dried to obtain 337 g of 5-amino-4-chloro-3-methyl-1H-pyrazole hydrochloride (B).

A pyrazoloazole coupler can be prepared from the compound (B), for example, according to the procedure as described in Example 3 at columns 30 to 31 of U.S. Patent 4,540,654.

Pyrazoloazole series couplers can also be synthesized from the 3-iminonitriles produced according to the present invention by, for example, a process shown as process (2) below, starting with the compound represented by the general formula (III). 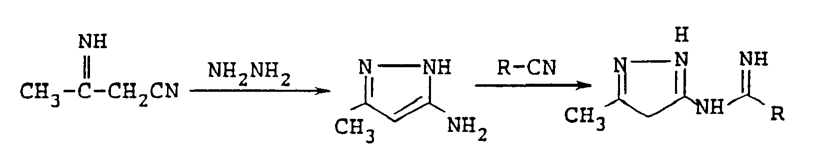 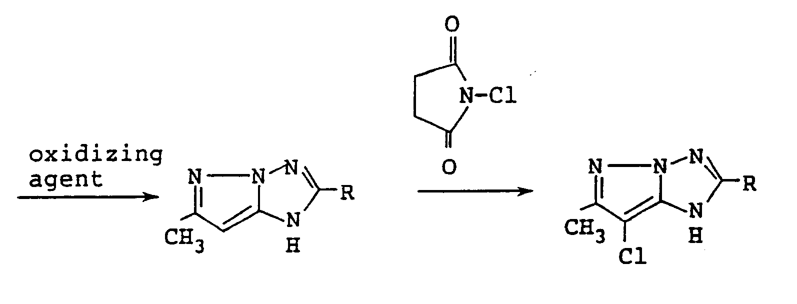
wherein R represents a substituent such as alkyl or aryl.

The process of the present invention enables one to synthesize 3-iminonitriles with more safety and more inexpensiveness than the conventional processes. Therefore, use of the 3-iminonitriles as intermediates for synthesizing photographic couplers or medicines and agricultural chemicals is feasible.

## Claims

1. A process for producing a 3-iminonitrile represented by the following general formula (II): 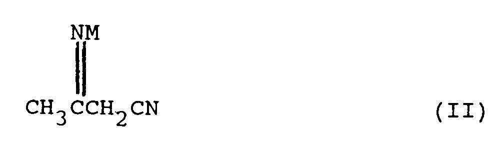 wherein M represents an alkali metal, which comprises reacting a compound of the formula (I):
CH₃CN (I)
with an alkali metal hydride.

2. The process of claim 1, wherein said alkali metal hydride is lithium hydride, sodium hydride or potassium hydride.

3. The process of claim 1, wherein said alkali metal hydride is used in an amount of from 0.5 to 5.0 molar equivalent based on the compound represented by formula (I).

4. The process of claim 1, wherein said reaction is conducted in the presence of a solvent for 30 min to 10h.

5. The process of claim 1 wherein said reaction is conducted at a temperature of from O^{o}C to 200^{o}C.

6. The process of claim 5 wherein said reaction is conducted at a temperature of from 20^{o}C to 150^{o}C.

7. A process for producing a 3-iminonitrile represented by the general formula (III): 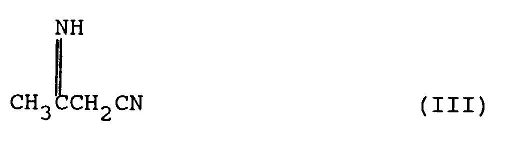 which comprises mixing the compound represented by the general formula 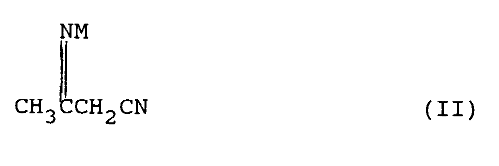 wherein M represents an alkali metal, with a protic solvent, wherein said compound represented by the general formula (II) is obtained by reacting a compound represented by the formula (I):
CH₃CN (I)
with an alkali metal hydride.

8. The process of claim 7 wherein said alkali metal hydride is lithium hydride, sodium hydride or potassium hydride.

9. The process of claim 7 wherein said protic solvent is used in an amount of at least 2 times as much as the amount of the alkali metal hydride used in molar ratio.

## Patentansprüche

1. Verfahren zum Herstellen von 3-Iminonitril, dargestellt durch die folgende allgemeine Formel (II): 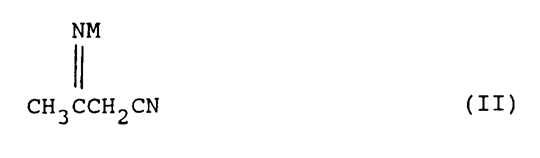 worin M ein Alkalimetall darstellt, welches das Reagierenlassen einer Verbindung der Formel (I):
CH₃CN (I)
mit einem Alkalimetallhydrid umfaßt.

2. Verfahren nach Anspruch 1, wobei das Alkalimetallhydrid Lithiumhydrid, Natriumhydrid oder Kaliumhydrid ist.

3. Verfahren nach Anspruch 1, wobei das Alkalimetallhydrid in einer Menge von 0,5 bis 5,0 moläquivalent, bezogen auf die Verbindung, dargestellt durch die Formel (I), verwendet wird.

4. Verfahren nach Anspruch 1, wobei die Reaktion in Anwesenheit eines Lösungsmittels 30 min bis 10 h durchgeführt wird.

5. Verfahren nach Anspruch 1, wobei die Reaktion bei einer Temperatur von 0^{o}C bis 200^{o}C durchgeführt wird.

6. Verfahren nach Anspruch 5, wobei die Reaktion bei einer Temperatur von 20^{o}C bis 150^{o}C durchgeführt wird.

7. Verfahren zum Herstellen von 3-Iminonitril, dargestellt durch die allgemeine Formel (III): 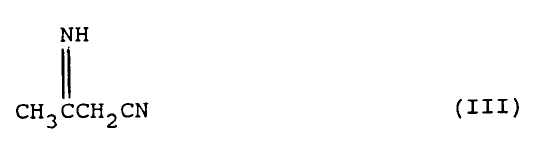 welches das Mischen der Verbindung, dargestellt durch die allgemeine Formel 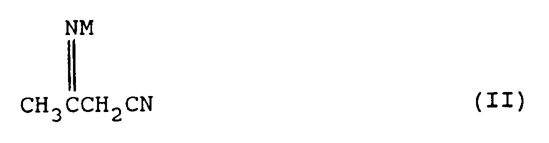 worin M ein Alkalimetall darstellt,
mit einem protischen Lösungsmittel umfaßt, wobei die Verbindung, dargestellt durch die allgemeine Formel (II) durch Reagierenlassen einer Verbindung, dargestellt durch die Formel (I):
CH₃CN (I)
mit einem Alkalimetallhydrid erhalten wird.

8. Verfahren nach Anspruch 7, wobei das Alkalimetallhydrid Lithiumhydrid, Natriumhydrid oder Kaliumhydrid ist.

9. Verfahren nach Anspruch 7, wobei das protische Lösungsmittel in einer Menge von mindestens dem 2-fachen der Menge des Alkalimetallhydrids, ausgedrückt als Molverhältnis, verwendet wird.

## Revendications

1. Procédé de production d'un 3-iminonitrile représenté par la formule générale suivante (II) : 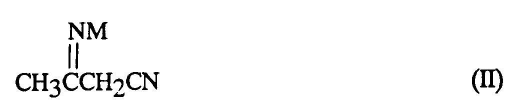 dans laquelle M représente un métal alcalin, qui comprend la réaction d'un composé de formule (I) :
CH₃CN (I)
avec un hydrure de métal alcalin.

2. Procédé selon la revendication 1, dans lequel ledit hydrure de métal alcalin est l'hydrure de lithium, l'hydrure de sodium ou l'hydrure de potassium.

3. Procédé selon la revendication 1, dans lequel ledit hydrure de métal alcalin est utilisé à raison de 0,5 à 5,0 équivalents molaires, par rapport au composé représenté par la formule (I).

4. Procédé selon la revendication 1, dans lequel ladite réaction est mise en oeuvre en présence d'un solvant pendant 30 min à 10 h.

5. Procédé selon la revendication 1, dans lequel ladite réaction est mise en oeuvre à une température comprise entre 0 et 200°C.

6. Procédé selon la revendication 5, dans lequel ladite réaction est mise en oeuvre à une température comprise entre 20 et 150°C.

7. Procédé de production d'un 3-iminonitrile représenté par la formule générale (III) : 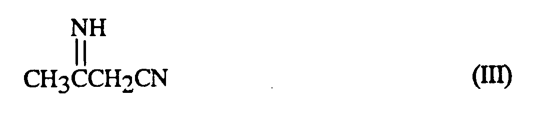 qui comprend le mélange du composé représenté par la formule générale : 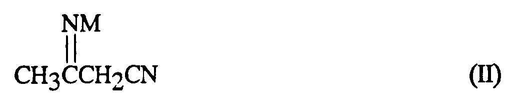 dans laquelle M représente un métal alcalin, avec un solvant protique, dans lequel ledit composé représenté par la formule générale (II) est obtenu en faisant réagir un composé représenté par la formule (I) :
CH₃CN (I)
avec un hydrure de métal alcalin.

8. Procédé selon la revendication 7, dans lequel ledit hydrure de métal alcalin est l'hydure de lithium, l'hydrure de sodium ou l'hydrure de potassium.

9. Procédé selon la revendication 7, dans lequel ledit solvant protique est utilisé en une quantité d'au moins deux fois la quantité de l'hydrure de métal alcalin utilisé, en rapport molaire.
